# EUROPEAN PATENT SPECIFICATION

(11) **EP 3 957 626 B1**
(45) Date of publication and mention of the grant of the patent: **15.11.2023**
(21) Application number: 21191678.8
(22) Date of filing: 17.08.2021
(51) Int. Cl.: C07C 225/16, A01N 33/12, A01N 55/00

(54) **REACTIVE QUATERNARY AMMONIUM ION-BASED ANTIMICROBIAL TREATMENTS FOR EXPOSED SURFACES**
ANTIMIKROBIELLE BEHANDLUNGEN BASIEREND AUF REAKTIVEN QUATERNÄREN AMMONIUMIONEN FÜR EXPONIERTE OBERFLÄCHEN
TRAITEMENTS ANTIMICROBIENS RÉACTIFS À BASE D'IONS D'AMMONIUM QUATERNAIRE POUR DES SURFACES EXPOSÉES

(30) Priority: 17.08.2020 US 202016995634
(43) Date of publication of application: 23.02.2022
(73) Proprietor: Goodrich Corporation, Charlotte, NC 28217-4578 (US)
(72) Inventor: HU, Jin, Hudson, OH, 44236 (US)
(74) Representative: Dehns

(56) References cited:
- E. POVERENOV ET. AL.: "Formation of Contact Active Antimicrobial Surfaces by Covalent Grafting of Quaternary Ammonium Compounds.", COLLOIDS AND SURFACES. B. BIOINTERFACES, vol. 169, 25 May 2018 (2018-05-25), pages 195-205, XP085417216, DOI: 10.1016/j.colsurfb.2018.04.065
- JING-GAO ET. AL.: "Surface Grafted Antimicrobial Polymer Networks with High Abrasion Resistance", ACS BIOMATERIALS SCIENCE AND ENGINEERING, vol. 2, no. 7, 30 May 2016 (2016-05-30), pages 1169-1179, XP055633609, DOI: 10.1021/acsbiomaterials.6b00221
- L. POROSA ET. AL.: "UV-Curable Contact Active Benzophenone Terminated Quaternary Ammonium Antimicrobials for Applications in Polymer Plastics and Related Devices.", APPLIED MATERIALS AND INTERFACES, vol. 9, no. 33, 4 August 2017 (2017-08-04) , pages 27491-27503, XP055851707, DOI: 10.1021/acsami/7b07363

## Description

### FIELD

The present disclosure relates to a composition and to products coated with the composition and, more particularly, to quaternary ammonium ion-based compositions and to products coated with such compositions to form products having antimicrobial treated surfaces.

### BACKGROUND

The recent novel-coronavirus (SARS-COV-2) outbreak has impacted the safety of passengers and crew members flying on aircraft. The safety of such passengers and crew members may be improved by treating surfaces - e.g., lavatory surfaces such as sinks or faucets - with antimicrobial treatments capable of mitigating the presence of the virus on such surfaces. Nano-silver based coatings or surface treatments are widely available and are very effective as antimicrobials as they suppress or inhibit a wide spectrum of bacteria, molds, fungi and viruses. Such coatings, however, are expensive and present significant environmental challenges.

Colloids and Surfaces. B Biointerfaces. Vol. 169, p. 195-205 (2018) XP085417216 relates to the formation of contact active antimicrobial surfaces by covalent grafting of quaternary ammonium compounds. ACS Biomaterials Science and Engineering, vol. 2, p. 1169-79 (2016) relates to UV-Curable contact active benzophenone terminated quaternary ammonium antimicrobials for applications in polymer plastics and related devices. Applied Materials and Interfaces, vol. 9 p. 27491-27503 (2017) relates to surface grafted antimicrobial polymer networks with high abrasion resistance.

### SUMMARY

A method of manufacturing a component of an aircraft interior having antimicrobial properties is disclosed. In various embodiments, the method includes applying a reactive quaternary ammonium ion-based compound ("reactive quat") to an exposed surface of the component.

The reactive quat comprises a reactive silane group, wherein the reactive quat is 1-tetradecanaminium, N,N-dimethyl-N-(3-(trimethoxysilyl)propyl)chloride, or 1-decanaminium, N-decyl-N-methyl-N-(3 (trimethoxysilyl)propyl)chloride, or 1-octadecanaminium, N,N-dimethyl-N-(3-(trihydroxysilyl)propyl)chloride; or the reactive quat comprises benzophenone. In various embodiments, the reactive quat comprises an alkyl chain. In various embodiments, the exposed surface comprises a metallic surface. In various embodiments, applying the reactive quat to the exposed surface comprises reacting the reactive silane group with the metallic surface.

[deleted]

In various embodiments, the reactive quat comprises an alkyl chain. In various embodiments, the exposed surface comprises a plastic surface. In various embodiments, applying the reactive quat to the exposed surface comprises reacting the benzophenone with the plastic surface. In various embodiments, applying the reactive quat to the exposed surface comprises exposing the benzophenone and the plastic surface to an ultraviolet radiation.

A component for an aircraft is disclosed according to claim 10. In various embodiments, the component includes an exposed surface; and an antimicrobial agent applied to the exposed surface, the antimicrobial agent comprising a reactive quat.

In various embodiments, the reactive quat comprises an alkyl chain. In various embodiments, the exposed surface comprises a metallic surface.

In various embodiments, the reactive quat comprises an alkyl chain. In various embodiments, the exposed surface comprises a plastic surface.

The foregoing features and elements may be combined in any combination, without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1 illustrates a chemical composition for a reactive quat, in accordance with various embodiments; and
FIG. 2 illustrates a surface portion of a component having a reactive quat-based surface coating applied thereon, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the invention as defined by the claims. Thus, the detailed description herein is presented for purposes of illustration only and not of limitation. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

Reactive quaternary ammonium ion-based compounds (referred to herein as "quats") have the ability to react and attach themselves to material surfaces and to form films or coatings for long lasting and non-leaching antimicrobial surface treatments. In this disclosure, reactive quats are proposed for antimicrobial treatments or coatings for exposed surfaces within an aircraft - *e.g.,* the surfaces of lavatory sinks or faucets or lavatory toilets, handles or lids or similar surfaces that are subject to exposure to viruses or pathogens transmitted, for example, by human touch or by airborne means. With respect to various embodiments, two representative reactive quats are described - a reactive silane quaternary ammonium ion-based antimicrobial and a benzophenone small molecule antimicrobial (BPAM). Both reactive quats are beneficial for surface treatments or coatings on surfaces within aircraft.

### Reactive Silane Quaternary Ammonium Ion-Based Quats.

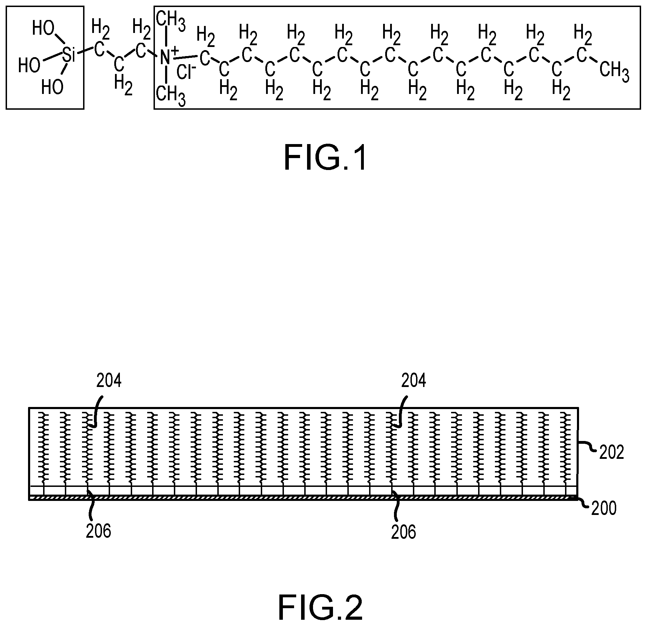

Typical silane quats include the following, which are functionalized with reactive alkoxy silane or hydroxylsilane: (i) 1-octadecanaminium, N,N-dimethyl-N-(3-(trimethoxsil)propyl)chloride; (ii) 1-tetradecanaminium, N,N-dimethyl-N-(3-(trimethoxysilyl)propyl)chloride; (iii) 1-decanaminium, N-decyl-N-methyl-N-(3 (trimethoxysilyl)propyl)chloride; and (iv) 1-octadecanaminium, N,N-dimethyl-N-(3-(trihydroxysilyl)propyl)chloride. The last of these reactive quats, 1-octadecanaminium, N,N-dimethyl-N-(3-(trihydroxysilyl)propyl)chloride, may be created *in situ* by combining the first of the reactive quats, 1-octadecanaminium, N,N-dimethyl-N-(3-(trimethoxsil)propyl)chloride, with water. Its chemical structure is illustrated in FIG. 1.

Referring to FIG. 1, the active ingredient of the compound comprises a quaternary ammonium ion as illustrated in the right-side box of FIG. 1. The positively charged nitrogen atom (N⁺) attracts negatively charged microbes, which are then destroyed or killed by the quat. More specifically, the proteins (or the spike proteins) on the outer surface of the SARS-COV-2 virus have been calculated to be negatively charged under neutral pH conditions. Thus, the SARS-COV-2 is electrically attracted to the quat. When attracted to the quat, the long molecular carbon or alkyl chain (also referred to as a spike or a sword) comes into contact with the offending microbe or virus. The carbon or alkyl chain then acts like a sword that punctures the outer membrane or coating of the microbe or virus coming in contact with it. For the SARS-COV-2 virus, the hydrophilic envelope surrounding the virus is penetrated by the carbon or alkyl chain, thereby terminating the viability of the virus. Still referring to FIG. 1, the reactive silane or sily group (RO)3Si- (R = CH3 or OH) of the quat is illustrated in the left-side box. The silane moieties covalently bind to surfaces such as metallic surfaces and textiles creating a transparent coating with antimicrobial properties or an antimicrobial agent. Further, since each silicon atom has three reactive groups, the spare reactive group after bonding to a surface can also bridge to neighboring silicon atoms in the compound, resulting in a robust antimicrobial film or coating on the surface.

Referring now to FIG. 2, a surface 200 having a reactive quat coating 202 is illustrated. As described above, the reactive quat coating 202 comprises a plurality of spikes 204 in the form of quaternary ammonium ions having long molecular carbon or alkyl chains, with each spike being attached to the surface 200 via a silane group 206. In various embodiments, the surface 200 may represent an outer surface (or an exposed surface) of various components found on an aircraft, such as, for example, a toilet, a toilet lid or handle, a sink or a sink faucet. Further applicable components on an aircraft include passenger seat fabrics or textiles, seat arms and meal trays. More generally, the surface 200 may comprise a siliceous surface, such as, for example, glass, glass wool, sand stone or ceramic surfaces. In various embodiments, the surface 200 may comprise natural fibers, such as, for example, cotton, wool, linen or felt surfaces. In various embodiments, the surface 200 may comprise man-made fibers, such as, for example, acrylic, modacrylic, polyester, cellulose acetate, rayon, acetate, anidex, spandex, vinyl, dacron or viscose surfaces. In various embodiments, the surface 200 may comprise metals, such as, for example, aluminum, stainless steel or galvanized metal surfaces. In various embodiments, the surface 200 may comprise miscellaneous other surfaces, such as, for example, leather, wood, rubber, plastic or formica surfaces.

Still referring to FIG. 2, in various embodiments, the reactive quat coating 202 is covalently bonded to the surface 200 (*e.g.,* a metallic surface), as described above. In various embodiments, e.g., where covalent bonding is not likely achievable because of the surface material, the reactive quat coating 202, may comprise a polymer-type coating or resin that includes a reactive quat compound as an additive. The polymer-type coating or resin may then be chemically adhered or bonded to the surface 200, thereby adhering or bonding the reactive quat compound to the surface as well. This allows the broad array of surface materials described above to be coated with reactive quat compounds, either through covalent bonding directly to the surface or by mixing of the compound within the coating itself. Various attributes of the reactive quat coatings described herein include resistance to organic solvents, resistance to strong acids or bases, resistance to leaching in water, salt or sweat solutions, thermal stability to upwards of 257 degrees Celsius (≈ 494° F), durability to upwards of one-hundred launderings or cleanings and durability to outlast the life of a product or component to which the coating is applied.

### Benzophenone Small Molecule Antimicrobial (BPAM) Quats.

The reactive silane group described above with reference to FIG. 1 react the Si(OMe) or SiOH with hydroxyl or (-OH) on metals, ceramics and some textiles, leather or plastics, such as cellulose, to obtain strong covalent bonding. But for some plastics or composites that lack hydroxyl groups on their surfaces (e.g., a plastic surface), the bonding may not be as strong as provided through covalent boding. In contrast, Benzophenone Small Molecule Antimicrobial (BPAM) quats are able to form strong chemical bonds to plastics and polymers via reaction with C-H groups (or methine groups) under mild ultraviolet radiation for a period of time (*e.g.,* one to five minutes). Thin coatings result, with the coatings including long molecular carbon or alkyl chains similar to those described above with reference to FIG. 1. These coatings share many of the properties of the coatings described above, and also exhibit robust abrasion resistance. In various embodiments, the BPAM quats may be represented by replacing the silane group illustrated in FIG. 1 with benzophenone, which has the chemical formula (C₆H₅)₂CO.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 10%, within 5%, within 1%, within 0.1%, or within 0.01% of a stated value. Additionally, the terms "substantially," "about" or "approximately" as used herein represent an amount close to the stated amount that still performs a desired function or achieves a desired result. For example, the term "substantially," "about" or "approximately" may refer to an amount that is within 10% of, within 5% of, within 1% of, within 0.1% of, and within 0.01% of a stated amount or value.

Finally, it should be understood that any of the above described concepts can be used alone or in combination with any or all of the other above described concepts. Although various embodiments have been disclosed and described, one of ordinary skill in this art would recognize that certain modifications would come within the scope of invention as defined by the claims.

## Claims

1. A method of manufacturing a component of an aircraft interior having antimicrobial properties, comprising:
applying a reactive quaternary ammonium ion-based compound ("reactive quat") to an exposed aircraft interior surface of the component; and; reacting the applied reactive quat with the exposed aircraft interior surface;
wherein the reactive quat comprises a reactive silane group, wherein the reactive quat is 1-tetradecanaminium, N,N-dimethyl-N-(3-(trimethoxysilyl)propyl)chloride, or wherein the reactive quat is 1-decanaminium, N-decyl-N-methyl-N-(3 (trimethoxysilyl)propyl)chloride, or wherein the reactive quat is 1-octadecanaminium, N,N-dimethyl-N-(3-(trihydroxysilyl)propyl)chloride;
or wherein the reactive quat comprises benzophenone.

2. The method of claim 1, wherein the reactive quat comprises an alkyl chain.

3. The method of claim 2, wherein the exposed aircraft interior surface comprises a metallic surface.

4. The method of claim 3, wherein applying the reactive quat to the exposed aircraft interior surface comprises reacting the reactive silane group with the metallic surface.

5. The method of any preceding claim, wherein the reactive quat comprises an alkyl chain.

6. The method of claim 5, wherein the exposed surface comprises a plastic surface.

7. The method of claim 6, wherein applying the reactive quat to the exposed surface comprises reacting the benzophenone with the plastic surface.

8. The method of claim 7, wherein applying the reactive quat to the exposed surface comprises exposing the benzophenone and the plastic surface to an ultraviolet radiation.

9. A component for an aircraft, comprising:
an exposed aircraft interior surface (200); and
an antimicrobial agent (202) applied to the exposed surface, the antimicrobial agent comprising a reactive quat; wherein the antimicrobial agent is reacted with the exposed aircraft interior surface;
wherein the reactive quat comprises a reactive silane group, wherein the reactive quat is 1-tetradecanaminium, N,N-dimethyl-N-(3-(trimethoxysilyl)propyl)chloride, or wherein the reactive quat is 1-decanaminium, N-decyl-N-methyl-N-(3 (trimethoxysilyl)propyl)chloride, or wherein the reactive quat is 1-octadecanaminium, N,N-dimethyl-N-(3 -(trihydroxysilyl)propyl)chloride; or
wherein the reactive quat comprises benzophenone.

10. The component of claim 9, wherein the reactive quat comprises an alkyl chain.

11. The component of claim 10, wherein the exposed surface comprises a metallic surface, or wherein the exposed surface comprises a plastic surface.

## Patentansprüche

1. Verfahren zum Herstellen eines Bauteils eines Luftfahrzeuginnenraums mit antimikrobiellen Eigenschaften, umfassend:
Aufbringen einer reaktiven Verbindung auf Basis quaternärer Ammoniumionen ("reaktives Quat") auf eine exponierte Luftfahrzeuginnenraumoberfläche des Bauteils; und; Reagieren des aufgebrachten reaktiven Quats mit der exponierten Luftfahrzeuginnenraumoberfläche;
wobei das reaktive Quat eine reaktive Silangruppe umfasst, wobei das reaktive Quat 1-Tetradecanaminium, N,N-Dimethyl-N-(3-(Trimethoxysilyl)propyl)chlorid ist, oder wobei das reaktive Quat 1-Decanaminium, N-Decyl-N-methyl-N-(3-(Trimethoxysilyl)propyl)chlorid ist, oder wobei das reaktive Quat 1-Octadecanaminium, N,N-Dimethyl-N-(3-(Trihydroxysilyl)propyl)chlorid ist;
oder wobei das reaktive Quat Benzophenon umfasst.

2. Verfahren nach Anspruch 1, wobei das reaktive Quat eine Alkylkette umfasst.

3. Verfahren nach Anspruch 2, wobei die exponierte Luftfahrzeuginnenraumoberfläche eine metallische Oberfläche umfasst.

4. Verfahren nach Anspruch 3, wobei das Aufbringen des reaktiven Quats auf die exponierte Luftfahrzeuginnenraumoberfläche das Reagieren der reaktiven Silangruppe mit der metallischen Oberfläche umfasst.

5. Verfahren nach einem der vorhergehenden Ansprüche, wobei das reaktive Quat eine Alkylkette umfasst.

6. Verfahren nach Anspruch 5, wobei die exponierte Oberfläche eine Kunststoffoberfläche umfasst.

7. Verfahren nach Anspruch 6, wobei das Aufbringen des reaktiven Quats auf die exponierte Oberfläche das Reagieren des Benzophenons mit der Kunststoffoberfläche umfasst.

8. Verfahren nach Anspruch 7, wobei das Aufbringen des reaktiven Quats auf die exponierte Oberfläche das Aussetzen des Benzophenons und der Kunststoffoberfläche einer ultravioletten Strahlung umfasst.

9. Bauteil für ein Luftfahrzeug, umfassend:
eine exponierte Luftfahrzeuginnenraumoberfläche (200); und
ein antimikrobielles Mittel (202), das auf die exponierte Oberfläche aufgebracht wird, wobei das antimikrobielle Mittel ein reaktives Quat umfasst; wobei das antimikrobielle Mittel mit der exponierten Luftfahrzeuginnenraumoberfläche zur Reaktion gebracht wird;
wobei das reaktive Quat eine reaktive Silangruppe umfasst, wobei das reaktive Quat 1-Tetradecanaminium, N,N-Dimethyl-N-(3-(Trimethoxysilyl)propyl)chlorid ist, oder wobei das reaktive Quat 1-Decanaminium, N-Decyl-N-methyl-N-(3-(Trimethoxysilyl)propyl)chlorid ist, oder wobei das reaktive Quat 1-Octadecanaminium, N,N-Dimethyl-N-(3-(Trihydroxysilyl)propyl)chlorid ist; oder
wobei das reaktive Quat Benzophenon umfasst.

10. Bauteil nach Anspruch 9, wobei das reaktive Quat eine Alkylkette umfasst.

11. Bauteil nach Anspruch 10, wobei die exponierte Oberfläche eine metallische Oberfläche umfasst oder wobei die exponierte Oberfläche eine Kunststoffoberfläche umfasst.

## Revendications

1. Procédé de fabrication d'un composant d'un intérieur d'aéronef présentant des propriétés antimicrobiennes, comprenant :
l'application d'un composé réactif à base d'ions d'ammonium quaternaire (« quat réactif ») sur une surface exposée d'intérieur d'aéronef du composant ; et ; la réaction du quat réactif appliqué avec la surface exposée d'intérieur d'aéronef ;
dans lequel le quat réactif comprend un groupe silane réactif, dans lequel le quat réactif est le chlorure de N,N-diméthyl-N-(3-(triméthoxysilyl)propyl)-1-tétradécanaminium, ou dans lequel le quat réactif est le chlorure de N-décyl-N-méthyl-N-(3-(triméthoxysilyl)propyl)-1-décanaminium, ou dans lequel le quat réactif est le chlorure de N,N-diméthyl-N-(3-(trihydroxysilyl)propyl)-1-octadécanaminium ;
ou dans lequel le quat réactif comprend de la benzophénone.

2. Procédé selon la revendication 1, dans lequel le quat réactif comprend une chaîne alkyle.

3. Procédé selon la revendication 2, dans lequel la surface exposée d'intérieur d'aéronef comprend une surface métallique.

4. Procédé selon la revendication 3, dans lequel l'application du quat réactif sur la surface exposée d'intérieur d'aéronef comprend la réaction du groupe silane réactif avec la surface métallique.

5. Procédé selon une quelconque revendication précédente, dans lequel le quat réactif comprend une chaîne alkyle.

6. Procédé selon la revendication 5, dans lequel la surface exposée comprend une surface en matière plastique.

7. Procédé selon la revendication 6, dans lequel l'application du quat réactif sur la surface exposée comprend la réaction de la benzophénone avec la surface en matière plastique.

8. Procédé selon la revendication 7, dans lequel l'application du quat réactif sur la surface exposée comprend l'exposition de la benzophénone et de la surface en matière plastique à un rayonnement ultraviolet.

9. Composant pour un aéronef, comprenant :
une surface exposée d'intérieur d'aéronef (200) ; et
un agent antimicrobien (202) appliqué sur la surface exposée, l'agent antimicrobien comprenant un quat réactif ; dans lequel l'agent antimicrobien réagit avec la surface exposée d'intérieur d'aéronef ;
dans lequel le quat réactif comprend un groupe silane réactif, dans lequel le quat réactif est le chlorure de N,N-diméthyl-N-(3-(triméthoxysilyl)propyl)-1-tétradécanaminium, ou dans lequel le quat réactif est le chlorure de N-décyl-N-méthyl-N-(3-(triméthoxysilyl)propyl)-1-décanaminium, ou dans lequel le quat réactif est le chlorure de N,N-diméthyl-N-(3-(trihydroxysilyl)propyl)-1-octadécanaminium ; ou
dans lequel le quat réactif comprend de la benzophénone.

10. Composant selon la revendication 9, dans lequel le quat réactif comprend une chaîne alkyle.

11. Composant selon la revendication 10, dans lequel la surface exposée comprend une surface métallique, ou dans lequel la surface exposée comprend une surface en matière plastique.
